# EUROPEAN PATENT APPLICATION

(11) **EP 1 374 869 A1**
(43) Date of publication of application: **02.01.2004**
(21) Application number: 02707292.5
(22) Date of filing: 02.04.2002
(51) Int. Cl.: A61K 31/506, A61P 3/06, A61P 3/10, A61P 13/12, A61P 43/00, C07D 239/47

(54) **NOVEL USE OF ARYLETHENE SULFONAMIDE DERIVATIVE**

(30) Priority: 03.04.2001 JP 2001103994
(71) Applicant: YAMANOUCHI PHARMACEUTICAL CO. LTD., Tokyo 103-8411 (JP)
(72) Inventor: FUJIMORI, Akira, c/o Yamanouchi Pharm. Co., Ltd, Tsukuba-shi, Ibaraki 305-8585 (JP); YUYAMA, Hironori, c/o Yamanouchi Pharm. Co., Ltd, Tsukuba-shi, Ibaraki 305-8585 (JP); TAHARA, Atsuo, c/o Yamanouchi Pharm. Co., Ltd, Tsukuba-shi, Ibaraki 305-8585 (JP); SONODA, Rie, c/o Yamanouchi Pharm. Co., Ltd, Tsukuba-shi, Ibaraki 305-8585 (JP); SHIBASAKI, Kumiko, c/o Yamanouchi Pharm. Co., Ltd, Tsukuba-shi, Ibaraki 305-8585 (JP)
(74) Representative: HOFFMANN EITLE
(86) International application number: PCT/JP2002/003289
(87) International publication number: WO 2002/080924

(57) **Abstract**

Novel use of N-[6-methoxy-5-(2-methoxyphenoxy)-2-(pyrimidin-2-yl)pyrimidin-4-yl]-2-phenylethenesulfonamide or a pharmaceutically acceptable salt thereof. Improvement of the following particular condition in a diabetic patient: (1) Elevation of blood glucose level; (2) Elevation of blood lipid level after the onset of early-stage nephropathy; (3) Renal dysfunction after the onset of early-stage nephropathy; (4) Increase of urinary albumin excretion after the onset of early-stage nephropathy; (5) Glomerular hyperfiltration after the onset of early-stage nephropathy; (6) Renal dysfunction after the progress toward chronic renal failure; (7) Increase of urinary protein excretion after the progress toward chronic renal failure.

## Description

### Technical Field of the Invention

The present invention relates to novel use of N-[6-methoxy-5-(2-methoxyphenoxy)-2-(pyrimidin-2-yl)pyrimidin-4-yl]-2-phenylethenesulfona mide or salts thereof.

### Background Art

Endothelin (hereinafter referred to as "ET") is an endogenous physiologically active peptide consisting of 21 amino acids, and known to exist as 3 types of iso-peptides, i.e., ET-1, ET-2 and ET-3, of which the amino acid sequences are slightly different each other. ET binds to the ET receptor on the target cellular membrane to exhibit a physiological activity. Up to now, as for the ET receptor, it is known that there are at least 2 sub-types, i.e., ET_{A} and ET_{B}. ET_{A} receptor has higher affinity to ET-1 and ET-2 than to ET-3, and ET_{B} receptor has the same degree of affinity to ET-1, ET-2 and ET-3.

N-[6-Methoxy-5-(2-methoxyphenoxy)-2-(pyrimidin-2-yl)pyrimidin-4-yl]-2-phenylethenesulfonamide (hereinafter referred to as "Compound A") or salts thereof have been disclosed in International Patent Publication No. 97/22595, in which their effect of inhibiting the binding of ET-1 to the ET_{A} receptor as well as the effect of inhibiting the ET-1-induced vasoconstriction and elevation of blood pressure have been disclosed specifically, but there is no disclosure on other particular effect. On the other hand, a number of diseases in which ET is possibly involved have been exemplified as follows: essential hypertension, pulmonary hypertension, erythropoietin-induced hypertension, cyclosporin A-induced hypertension, bronchial asthma, acute renal failure, chronic renal failure, glomerular nephritis, cyclosporin-induced renal failure, acute myocardial infarction, unstable angina pectoris, chronic heart failure, cerebrovascular spasm mainly caused by subarachnoid hemorrhage, cerebral ischemic disturbance, urinary incontinence, benign prostatic hypertrophy, arteriosclerosis, Raynaud's syndrome, diabetic peripheral circulatory disturbance, diabetic nephropathy, preeclampsia, premature labor, peptic ulcer, hepatic insufficiency, rheumatism, restenosis after PTCA, chronic respiratory failure, chronic obstructive pulmonary disease, cor pulmonale, acute respiratory failure, pulmonary edema, ischemic hepatopathy, adult respiratory distress syndrome, interstitial pneumonia, fibroid lung, glaucoma, osteoarthritis, chronic rheumatoid arthritis, liver cirrhosis, inflammatory enteropathy, cancer, and the like.

### Disclosure of the Invention

The present inventors worked assiduously to elucidate therapeutic possibility of Compound A or salts thereof for another specific disease in order to create a novel drug. As a result, the inventors have unexpectedly found that Compound A or salts thereof can improve the following specific conditions in diabetes mellitus which have not specifically disclosed in the above-mentioned International Patent Publication No. 97/22595. That is, (1) elevation of blood glucose level, (2) elevation of blood lipid level following the complication of early-stage nephropathy, (3) renal dysfunction following the complication of early-stage nephropathy, (4) increase of urinary albumin excretion following the complication of early-stage nephropathy, (5) glomerular hyperfiltration following the complication of early-stage nephropathy, (6) renal dysfunction following the change into chronic renal failure, and (7) increase of urinary protein excretion following the change into chronic renal failure.

More particularly, Compound A or salts thereof, as shown in Test Example 1 described below in rats of diabetes mellitus induced by streptozotocin (STZ), showed a significantly improved effect on a significant elevation of blood glucose level, an elevation of blood cholesterol level and an elevation of blood triglyceride level induced by administration of STZ at 1 mg/kg/day, p.o. The elevation of the blood cholesterol level or triglyceride level is frequently observed as a result of leakage of proteins such as albumin caused by renal dysfunction at the onset of a complication of the early-stage nephropathy in diabetes mellitus.

The improvement of this kind, which has not been disclosed or suggested in the above-mentioned International Patent Publication No. 97/22595, is a novel action of Compound A or salts thereof found by the present inventors for the first time. In addition, it has been reported on FR139317, a linear tripeptide with ET_{A} receptor antagonism, and on bosentan, a non-peptide ET_{A/B} receptor antagonist, that they do not change the plasma glucose level in STZ-induced diabetic rats (Progress in Medicine, 15 (10), 2108, 1995; Kidney Int., 57 (5), 1882, 2000). It has also been reported that darusentan, a non-peptide ET_{A} receptor inhibitor, decreases the blood triglyceride level at 100 mg/kg, p.o. (in STZ-induced diabetic rats, however, the triglyceride level is not elevated but decreased only in comparison with that of a control group), but it does not change the blood glucose level (J. Pharm. Exp. Ther., 293 (2), 351, 2000). In other words, in the ET receptor antagonists, there is no report on improvement of the blood glucose elevation in diabetes mellitus and on improvement of the blood lipid elevation following the onset of complication of the early nephropathy in diabetes mellitus.

In addition, Compound A or salts thereof, as shown in Test Example 1 described below in rats of diabetes mellitus induced by STZ, showed a significantly improved effect on a significant increase of urinary albumin excretion by administration of STZ at 1 mg/kg/day, p.o., and a significant tendency of improvement on the glomerular hyperfiltration in administration of STZ, i.e., improvement of renal dysfunction. Moreover, Compound A or salts thereof, as shown in Test Example 2 described below in 5/6 nephrectomized rats as a model animal of chronic renal failure, showed a significant improvement at 1 mg/kg/day, p.o., on significant increase of the urinary protein excretion without giving any influence on blood pressure, i.e., improvement of renal dysfunction.

As some of a large number of expected diseases described in the above-mentioned International Patent Publication No. 97/22595, "diabetic nephropathy", "chronic renal failure" and "acute renal failure" are specifically exemplified concerning the improvement of renal dysfunction in diabetes mellitus accompanied by the above-mentioned early nephropathy or advanced to chronic renal failure, but there is no evidence for them or there is no description on improvement of increase of the urinary albumin excretion, improvement of the glomerular hyperfiltration, and improvement of increase of the urinary protein excretion. Further, there is a report that the above mentioned peptide FR139317 when continuously injected intraperitoneally reduces excretion of microalbuminuria and inhibits gene expression of the extracellular matrix of the glomerulus in STZ-induced diabetic rats (Diabetes, 44, 895-899, 1995). With respect to orally administrable non-peptide ET receptor antagonists, however, only one report on the above-mentioned darusentan has been published indicating that it has a tendency to reduce microalbuminuria in STZ-induced diabetic rats at a dose of 100 mg/kg/day for 160 days, the dose being much higher than that of the effective component of the invention (J. Cardiovasc. Pharmacol., 31 (Suppl 1), S492-S495, 1998). Chronic renal failure is considered to be a final clinical state of diabetic nephropathy, a progressive chronic renal disease, which advances from diabetic nephropathy to chronic renal failure and finally to the term of dialysis therapy (Zin to Toseki (Kidney and Dialysis), 41, Manual for Therapy of Renal Diseases, 191-193, 1996, special issue). However, there is no report disclosing that orally administered ET_{A} receptor antagonists are effective in improving increase of the urinary protein excretion in chronic renal failure.

In addition, Compound A or salts thereof, as shown in Test Example 3 described below, did not change the systemic blood pressure in spontaneously hypertensive rats (SHR).

As a current therapy for diabetic nephropathy, a hypotensive therapy with ACE inhibitors or calcium antagonists has been conducted, but a hypotensive drug influencing on the systemic hemodynamics may sometimes decrease renal perfusion pressure with decrease of blood pressure. Particularly, in a case of renal dysfunction, it is suggested that occurrence of acute renal failure caused by an ACE inhibitor should be considered (Saishin Igaku (the Newest Medical Science), 48 (9), 84-90, 1993; Chiryogaku (Therapeutics), 30 (8), 27-32, 1996). In addition, though the usefulness of ACE inhibitors in pharmacotherapy during a resting phase of chronic renal failure has been recognized in an animal experiment, a prescription of an ACE inhibitor for the renal failure at the end stage is restricted since the administration of the ACE inhibitor has to be made carefully particularly in the case of renal dysfunction. In this connection, two death cases were observed in the group of renal dysfunction to which an ACE inhibitor, enalapril, was administered as shown in Test Example 2 mentioned below, but there was no death case in the group to which the effective component of the invention was administered.

In diabetic patients or model animals, production of ET-1 is enhanced (Diabetologia, 33, 306-310, 1990; J. Lab. Clin. Med., 122, 149-156, 1993), and there is a correlation between the severity of nephropathy and the blood ET-1 level (Jpn. J. Nephrol., 38 (Suppl 1), 141, 1996). Further, it has been reported that ET-1 is produced in various sites of kidney, such as endothelial cells including renal vasculature, glomerulus and collecting ducts (Kidney Int., 45, 336-344, 1994), contracts the renal arterioles (afferent vessel and efferent vessel), causes contraction or growth of glomerular mesangial cells with accumulation of extracellular matrix (Kekkan to Naihi (Blood vessel and Endothelium), 2 (3), 297-304, 1992; J. Clin. Invest., 83, 708-712, 1989), and inhibits reabsorption of sodium and water in the renal tubules (Igaku-no Ayumi (Proceedings of Medical Science), 170 (5), 393-396, 1994). The growth of glomerular mesangial cells indicates the preceding stage of the expansion of the mesangial region toward glomerular sclerosis, which is a cause resulting in the final renal dysfunction. Compound A or salts thereof, as shown in Test Example 4 described below, showed the effect of inhibiting the growth of rat's glomerular mesangial cells induced by ET-1, that is, Compound A or salts thereof improved ET-induced renal dysfunction accompanied by matrical change, for example, diabetic nephropathy, ET-induced early-stage nephropathy, and chronic renal failure accompanied by glomerular sclerosis which is considered as the final clinical state of complications of diabetes mellitus.

According to the invention, a pharmaceutical composition for improving at least one specific condition selected from the following items (1) to (7) in diabetic patients, which comprises Compound A or a pharmaceutically acceptable salt thereof as effective component, is provided.
(1) Elevation of blood glucose level
(2) Elevation of blood lipid level after the onset of early-stage nephropathy
(3) Renal dysfunction after the onset of early-stage nephropathy
(4) Increase of urinary albumin excretion after the onset of early-stage nephropathy
(5) Glomerular hyperfiltration after the onset of early-stage nephropathy
(6) Renal dysfunction after the progress toward chronic renal failure
(7) Increase of urinary protein excretion after the progress toward chronic renal failure

More particularly, the invention provides the following compositions comprising Compound A or a pharmaceutically acceptable salt thereof as effective component:
A pharmaceutical composition for improving elevation of the blood glucose level in a diabetic patient;
A pharmaceutical composition for improving elevation of the blood lipid level in a diabetic patient after the onset of early-stage nephropathy, a pharmaceutical composition for improving renal dysfunction, a pharmaceutical composition for improving renal dysfunction being a pharmaceutical composition for improving increase of urinary albumin excretion, and/or a pharmaceutical composition for improving renal dysfunction being a pharmaceutical composition for improving glomerular hyperfiltration;
A pharmaceutical composition for improving renal dysfunction in a diabetic patient after the progress toward chronic renal failure and/or a pharmaceutical composition for improving renal dysfunction being a pharmaceutical composition for improving increase of urinary protein excretion;
A pharmaceutical composition for improving renal dysfunction in a diabetic nephropathy patient, a pharmaceutical composition for improving renal dysfunction being a pharmaceutical composition for improving increase of urinary albumin excretion, and/or a pharmaceutical composition for improving renal dysfunction being a pharmaceutical composition for improving glomerular hyperfiltration;
A pharmaceutical composition for improving renal dysfunction in an ET-induced early nephropathy patient, a pharmaceutical composition for improving renal dysfunction being a pharmaceutical composition for improving increase of urinary albumin excretion, and/or a pharmaceutical composition for improving renal dysfunction being a pharmaceutical composition for improving glomerular hyperfiltration; and
A pharmaceutical composition for improving renal dysfunction in a chronic nephropathy patient and/or a pharmaceutical composition for improving renal dysfunction being a pharmaceutical composition for improving increase of urinary protein excretion.

The invention also relates to the use of Compound A or a pharmaceutically acceptable salt thereof for the purpose of producing an agent for improving at least one specific condition selected from the above items (1) to (7) in diabetic patients.

More particularly, the invention relates to the use of Compound A or a pharmaceutically acceptable salt thereof for the purpose of producing the following agents:
An agent for improving elevation of the blood glucose level in a diabetic patient;
An agent for improving elevation of the blood lipid level in a diabetic patient after the onset of early nephropathy, an agent for improving renal dysfunction, an agent for improving renal dysfunction being an agent for improving increase of urinary albumin excretion, and/or an agent for improving renal dysfunction being an agent for improving glomerular hyperfiltration;
An agent for improving renal dysfunction in a diabetic patient after the progress toward chronic renal failure and/or an agent for improving renal dysfunction being an agent for improving increase of urinary protein excretion;
An agent for improving renal dysfunction in a diabetic nephropathy patient, an agent for improving renal dysfunction being an agent for improving increase of urinary albumin excretion, and/or an agent for improving renal dysfunction being an agent for improving glomerular hyperfiltration;
An agent for improving renal dysfunction in an ET-induced early-stage nephropathy patient, an agent for improving renal dysfunction being an agent for improving increase of urinary albumin excretion, and/or an agent for improving renal dysfunction being an agent for improving glomerular hyperfiltration; and
An agent for improving renal dysfunction in a chronic nephropathy patient and/or an agent for improving renal dysfunction being an agent for improving increase of urinary protein excretion.

The invention also provides a method for improving at least one specific condition selected from the above items (1) to (7) in a diabetic patient, which comprises administering a therapeutically effective amount of Compound A or a pharmaceutically acceptable salt thereof to the patient.

More particularly, the invention provides the following methods which comprise administering a therapeutically effective amount of Compound A or a pharmaceutically acceptable salt thereof to a patient:
A method for improving elevation of the blood glucose level in a diabetic patient;
A method for improving elevation of the blood lipid level in a diabetic patient after the onset of early-stage nephropathy, a method for improving renal dysfunction, a method for improving renal dysfunction being a method for improving increase of urinary albumin excretion, and/or a method for improving renal dysfunction being a method for improving glomerular hyperfiltration;
A method for improving renal dysfunction in a diabetic patient after the progress toward chronic renal failure and/or a method for improving renal dysfunction being a method for improving increase of urinary protein excretion;
A method for improving renal dysfunction in a diabetic nephropathy patient, a method for improving renal dysfunction being a method for improving increase of urinary albumin excretion, and/or a method for improving renal dysfunction being a method for improving glomerular hyperfiltration;
A method for improving renal dysfunction in an ET-induced early nephropathy patient, a method for improving renal dysfunction being a method for improving increase of urinary albumin excretion, and/or a method for improving renal dysfunction being a method for improving glomerular hyperfiltration; and
A method for improving renal dysfunction in a chronic nephropathy patient and/or a method for improving renal dysfunction being a method for improving increase of urinary protein excretion.

A current hypotensive therapy conducted for diabetic nephropathy has an influence on the systemic hemodynamics and is in danger of decreasing renal perfusion pressure with decrease of blood pressure. On the contrary, the effective component of the invention acts in such a degree that almost no systemic blood pressure is changed and therefore it can be administered to such a risky patient. Moreover, the effective component of the invention may be an excellent oral therapeutic agent since it is well absorbed in oral administration.

The invention will be explained in more details as follows.

According to the 1991 research study report on diabetes mellitus edited by Shigeta et al. under the Ministry of Health and Welfare, the nephropathy can be classified into 5 categories depending on the degree of progress, i.e., 1st stage (pre-stage of nephropathy), 2nd stage (early stage of nephropathy), 3rd stage (manifest stage of nephropathy), 4th stage (stage of renal failure), and 5th stage (stage of dialysis therapy). In this specification, accordingly, a "diabetic patient following the onset of early-stage nephropathy" indicate the patient whose complicated nephropathy is at or after the 2nd stage; a "diabetic patient after the progress toward chronic renal failure" indicate the patient whose complicated nephropathy is at or after the 4th stage; a "patient with ET-induced early-stage nephropathy" indicates the patient whose nephropathy is caused by the enhancement of ET production in diabetes mellitus etc. and is at or after the 2nd stage.

In this specification, the phrase "no systemic blood pressure is changed" indicates that the change of the systemic blood pressure is in such a degree that there is no danger of decrease of blood pressure and of renal perfusion pressure, though a current therapy for lowering the blood pressure conducted for diabetic nephropathy has an influence on the systemic hemodynamics and may cause such danger. Therefore, "a pharmaceutical composition for improving renal dysfunction by which no systemic blood pressure is changed" indicates such a composition that can be administered to a patient for whom administration of an ACE inhibitor etc. is inhibited or hesitated because there is a possibility to cause acute renal failure such as renal dysfunction.

The pharmaceutically effective component of the invention is Compound A or a pharmaceutically acceptable salt thereof. Such a salt is exemplified by those described in the above-mentioned International Patent Publication No. 97/22595, specifically including an acid addition salt with an inorganic acid such as hydrochloric acid, hydrobromic acid, hydroiodic acid, sulfuric acid; nitric acid or phosphoric acid, or organic acid such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, aspartic acid or glutamic acid; salt with an inorganic base such as sodium, potassium, magnesium, calcium or aluminum, or organic base such as methylamine, ethylamine, ethanolamine, lysine or ornithine; and ammonium salt. Particularly preferred is the potassium salt.

In addition, the effective component of the invention includes a variety of isomers, their mixtures, isolated products, hydrates, and solvates. In some cases, the effective component of the invention forms polymorphic crystals, all of which are included in the invention.

These compounds can easily be obtained according to the production process as described in the above-mentioned International Patent Publication No. 97/22595 or a similar process based thereon.

The pharmaceutical preparation of the invention may be made into an oral solid preparation, oral liquid preparation or injection according to a conventional way using orally or parenterally applicable organic or inorganic carriers, fillers, and other excipients. Since the pharmaceutically effective component of the invention is excellent in oral absorbability, the pharmaceutical preparation of the invention is suitable for oral preparations. The most preferred preparation is an oral solid preparation, which can easily be carried, preserved and taken by a patient per se.

As the oral solid preparation, tablets, powder, fine granules, granules, capsules, pills and sustained release preparations can be used. Such solid preparations may contain one or more of the active substances together with at least one of inert diluents such as lactose, mannitol, glucose, hydroxypropyl cellulose, microcrystalline cellulose, starch, cornstarch, polyvinylpyrrolidone, and magnesium metasilicate aluminate. The composition may contain other excipients than diluents according to a conventional way, for example, binders such as hydroxypropyl cellulose, hydroxypropyl methyl cellulose; lubricants such as magnesium stearate, polyethylene glycol, starch, talc; disintegrators such as fibrin calcium glycolate, carmellose calcium; stabilizers such as lactose; solubilizing agents such as glutamic acid or aspartic acid; plasticizers such as polyethylene glycol; coloring agents such as titanium oxide, talc, yellow iron oxide. The tablets or pills if required may be coated with a sugar-coating or gastro-coating or enteric coating film which is composed of saccharose, gelatine, agar, pectin, hydroxypropyl cellulose or hydroxyprophyl methyl cellulose phthalate.

The oral liquid preparation includes pharmaceutically acceptable emulsion, solution, suspension, syrup, and elixir, and may contain a generally used inert diluent, for example, purified water, ethanol. In addition to the inert diluent, the composition may contain an auxiliary agent such as wetting agent or suspending agent, sweetening agent, flavor, fragrance, preservative, and so on.

The injections for intravenous, intramuscular or subcutaneous injection include sterilized aqueous or non-aqueous solutions, suspensions and emulsions. As diluents for the aqueous solution or suspension, for example, distilled water for injection and physiological saline are included. As diluents for the non-aqueous solution or suspension, for example, propylene glycol, polyethylene glycol, a vegetable oil such as olive oil, an alcohol such as ethanol, and polysorbate 80 are included. Such a composition may further contain auxiliary agents, for example, a preservative, wetting agent, emulsifying agent, dispersant, stabilizer such as lactose, and solubilizing co-agent such as glutamic acid or aspartic acid. These may be sterilized by filtration through a bacteria-protecting filter, addition of a bactericide, or irradiation. These may also be formed into a sterilized solid composition, which may be dissolved in sterilized water or sterilized solvent for injection before use.

The dosage of the effective component of the invention may be determined properly according to the individual conditions considering the route of administration, condition of disease, age of the subject, gender, and so on. In general, the oral preparation may be administered at a dose of about 0.1-100 mg/day for an adult, preferably 1-20 mg/day, in a single or two divided doses.

The pharmaceutical preparation of the invention may be used simultaneously or separately after an interval in combination with another drug used in treatment of diabetes mellitus. Such a drug used together with the active component of the invention includes, for example, insulin preparations such as insulin, neutral insulin, aqueous suspension of non-crystalline insulin zinc, two-phase isophane insulin aqueous suspension, isophane insulin aqueous suspension, insulin zinc aqueous suspension, protamine insulin zinc aqueous suspension, and crystalline insulin zinc aqueous suspension; drugs of sulfonylurea type such as tolbutamide, glyclopyramide, acetohexamide, tolazamide, chlorpropamide, glibenclamide, and gliclazide; drugs of sulfonamide type such as glybuzole; drugs of biguanide type such as metformin hydrochloride, buformin hydrochloride; aldose reductase inhibitors such as epalrestat; α-glucosidase inhibitors such as voglibose, acarbose; insulin resistance improvers such as troglitazone, pioglitazone; somatomedin C preparations such as mecasermin; other hypoglycemic drugs such as nateglinide; prostanoid relating drugs such as beraprost, alprostadil alphadex, lipoPGE₁, lipoprost alphadex; ACE inhibitors such as enalapril maleate; Chinese drugs such as Gosya-jinnkigan; angiotensin II receptor antagonists; thromboxane synthetase inhibitors; vitamin B preparations; calcium blockers; and diuretics.

### Brief Description of the Drawings

Fig.1 is a graph showing the effect of a potassium salt of compound A (hereinafter referred to as "Compound 1") on the mean blood pressure and the heart rate in spontaneously hypertensive rats (SHR).
Fig.2 is a graph showing the effect of promoting the growth of rat's cultured mesangial cells by ET-1, ET-3 and sarafotoxin S6c (hereinafter referred to as "S6c").
Fig.3 is a graph showing the effect of promoting the hypertrophy of rat's cultured mesangial cells by ET-1, ET-3 and S6c.

### Best Mode for Carrying Out the Invention

The invention will be explained in more details based on Examples and Test Examples, which are not intended to restrict the invention. Compound 1 used in Examples and Test Examples mentioned below is the potassium salt of Compound A.

### Example 1: Capsules

**(Table 1)**

| Component | 2mg Capsule | 5mg Capsule | 10mg Capsule |
|---|---|---|---|
| Compound 1 | 2.0 mg | 5.0 mg | 10.0 mg |
| Lactose | 298.0 mg | 295.0 mg | 290.0 mg |
| Total | 300.0 mg | 300.0 mg | 300.0 mg |

The components as shown in Table 1 are mixed and filled in a capsule to give a capsule preparation.

### Test Example 1: Effect on the blood parameter and urinary parameter in diabetic rats induced by streptozotocin (STZ)

### (Method)

Wistar (Crj) rats of 9 weeks of age were divided into 5 groups so that there was no bias in urinary albumin excretion. To the 4 groups of the rats was administered intravenously STZ (50 mg/kg). Over 6 weeks after administration of STZ, only a solvent (0.5% methylcellulose solution (hereinafter abbreviated to 0.5%MC) was orally administered to the control group (STZ-Cont.) once a day continuously. Similarly, 0.1 mg/kg of Compound 1 was administered to the low dose group (STZ-Low Dose(0.1)), 1 mg/kg of Compound 1 to the high dose group (STZ-High Dose(1)), and 10 mg/kg of enalapril maleate to the group (STZ-Ena.) to which an ACE inhibitor had been administered. To the remaining one group was intravenously administered physiological saline, and then orally administered a solvent (0.5%MC) as a control group (Sham) continuously. After the lapse of 2, 4 and 6 weeks from the start of administration, urine was collected for 24 hours in a metabolic cage to measure a urinary parameter. The blood was collected from the tail vein after 2 and 4 weeks and from the abdominal aorta under anesthesia after 6 weeks to measure a blood parameter.

The results were indicated by the mean ± error. In comparison between the Sham group and the STZ-Cont. group, a t-test was made without correspondence, and when the significant level is less than 5%, it is considered as a significant difference. In comparison between the STZ-dose groups of rats, multiple comparison (Dunnett's test) was made respectively without correspondence following the variance analysis of one-way classification. When the significant level is less than 5%, it is considered as a significant difference.

### (Result)

### (1) Effect on the blood parameter in STZ-induced diabetic rats

### 1) Blood glucose

**(Table 2)**

| | Blood glucose level (mg/dl) | | |
|---|---|---|---|
| | 2 weeks | 4 weeks | 6 weeks |
| Sham (n=13) | 94.4±2.3 | 98.0±3.6 | 120.2±2.2 |
| STZ-Cont. (n=12) | 357.4±10.5 ** | 390.7±20.6** | 460.7±18.9** |
| STZ-Low Dose(0.1) (n=11-13) | 357.1±14.5 | 396.0±19.4 | 417.8±14.6 |
| STZ-High Dose(1) (n=11) | 381.7±12.9 | 376.2±19.6 | 367.1±12.2^{##} |
| STZ-Ena. (n=11) | 403.0±13.0 | 393.2±17.6 | 394.3±9.6^{##} |

| | | | |
|---|---|---|---|
| ** p<0.01 vs Sham group; | | | |
| ^{##} p<0.01 vs STZ-Cont. group | | | |

As shown in Table 2, significant elevation of the blood glucose level was observed from the 2nd week after the administration of STZ. In the STZ-High Dose (1) group, decrease of the same blood glucose level as that in the STZ-Ena. group was observed at a very low dose of 1 mg/kg after the lapse of 6 weeks from the start of administration.

It was suggested, accordingly, that Compound 1 has the effect of improving elevation of the blood glucose level in diabetes mellitus.

### 2) Blood lipid

**(Table 3)**

| | Blood cholesterol level (mg/dl) (4 weeks) |
|---|---|
| Sham (n=13) | 59.2±2.4 |
| STZ-Cont. (n=12) | 235.7±23.0** |
| STZ-Low Dose(0.1)(n=11) | 247.b±29.8 |
| STZ-High Dose(1)(n=10) | 158.1±14.7^{#} |
| STZ-Ena. (n=11) | 148.8±11.2^{#} |

| | |
|---|---|
| ** p<0.01 vs Sham group; | |
| ^{#} p<0.05 vs STZ-Cont. group | |

**(Table 4)**

| | Blood triglyceride level (mg/dl) (4 weeks) |
|---|---|
| Sham (n=13) | 157.8±13.0 |
| STZ-Cont. (n=12) | 729.0±89.3** |
| STZ-Low Dose(0.1)(n=11) | 866.6±97.5 |
| STZ-High Dose(1)(n=11) | 494.0±55.8^{#} |
| STZ-Ena. (n=11) | 407.0±35.7^{#} |

| | |
|---|---|
| ** p<0.01 vs Sham group; | |
| ^{#} p<0.05 vs STZ-Cont. group | |

As shown in Table 3 and Table 4, significant elevation of the blood cholesterol level and blood triglyceride level was observed from the 4th week after the administration of STZ. In the STZ-High Dose (1) group, decrease of the same blood cholesterol and triglyceride level as that in the STZ-Ena. group was observed at a very low dose of 1 mg/kg.

The elevation of the blood cholesterol level and blood triglyceride level suggests that the renal function is suffering from ome disturbance. Since the above mentioned model is considered as a pathological model of diabetes mellitus after the onset of complicated early nephropathy, it is suggested that Compound 1 has the effect of improving elevation of the blood lipid level in diabetes mellitus after the onset of complicated early-stage nephropathy.

### (2) Effect on the urinary parameter in STZ-induced diabetic rats

### 1) Urinary albumin

**(Table 5)**

| | Urinary albumin excretion (mg/day) | | | |
|---|---|---|---|---|
| | 0 week | 2 weeks | 4 weeks | 6 weeks |
| Sham (n=13) | 0.52±0.05 | 0.65±0.04 | 0.42±0.04 | 0.43±0.04 |
| STZ-Cont. (n=12-13) | 0.43±0.04 | 0.96±0.08* | 1.11±0.31* | 1.43±0.48* |
| STZ-Low Dose(0.1)(n=11-13) | 0.45±0.03 | 1.03±0.07 | 0.74±0.13 | 1.37±0.19 |
| STZ-High Dose(1)(n=10-13) | 0.50±0.05 | 0.67±0.05^{#} | 0.46±0.05^{#} | 1.03±0.12 |
| STZ-Ena. (n=9-13) | 0.46±0.04 | 0.91±0.13 | 0.65±0.14 | 1.27±0.18 |

| | | | | |
|---|---|---|---|---|
| * p<0.05 vs Sham group; | | | | |
| ^{#} p<0.05 vs STZ-Cont. group | | | | |

As shown in Table 5, significant increase of the urinary albumin excretion (UAE) was observed from the 2nd week after the administration of STZ. Compound 1 made UAE decrease dose-dependently after the lapse of 2, 4 and 6 weeks from the administration. On the other hand, no UAE reduction was observed in the STZ-Ena. group.

Therefore, it was suggested that Compound 1 has the effect of improving the increase of urinary albumin excretion after the onset of early nephropathy in diabetes mellitus.

### 2) Creatinine clearance

**(Table 6)**

| | Creatinine clearance (Uday) |
|---|---|
| Sham (n=13) | 1.8±0.2 |
| STZ-Cont. (n=12) | 2.6±0.2* |
| STZ-Low Dose(0.1)(n=13) | 2.9±0.5 |
| STZ-High Dose(1)(n=11) | 1.8±0.2 |
| STZ-Ena. (n=11) | 3.0±0.8 |

| | |
|---|---|
| * p<0.05 vs Sham group | |

As shown in Table 6, significant elevation of creatinine clearance (Ccr) was observed at the 2nd week after the administration of STZ, indicating that glomerular hyperfiltration occurred. After the lapse of 4 weeks, no marked elevation of Ccr was observed. In the STZ-High Dose(1) group, there was a tendency that elevation of Ccr after 2 weeks from the start of administration was inhibited. On the other hand, in the STZ-Ena. group, there was no tendency that elevation of Ccr was inhibited.

Therefore, it was suggested that Compound 1 has the effect of improving the glomerular hyperfiltration in diabetes mellitus after the onset of early-stage nephropathy.

### Test Example 2: Effect on the renal function in chronic renal failure (5/6Nx) rats induced by 5/6 nephrectomy

### (Method)

Male Wistar rats of 8 weeks of age were used in the experiment. A Rat was anesthetized with petobarbital and 2/3 of the left kidney was cut off, and one week after the right kidney was removed completely. Two weeks after the 5/6 nephrectomy, systolic blood pressure (SBP) was measured by a tail-cuff method, and the urine was collected in a metabolic cage for 24 hours to measure the urinary protein excretion. Rats were divided into 3 groups (each group: n=9) so that no difference occurred between the respective groups. Thereafter, a solvent (0.5%MC) was orally administered to the 5/6Nx-Cont. group at a dose of 5 ml/kg over 8 weeks. To the 5/6Nx-Compound 1 group was orally administered 1 mg/kg/day of Compound 1 dissolved in a solvent (0.5%MC) once a day continuously. Similarly, to the 5/6Nx-Ena. group was administered 10 mg/kg/day of enalapril maleate dissolved in a solvent (0.5%MC). On the other hand, the solvent (0.5%MC) alone was orally administered to the abdominal-sectioned rats as a control group (Sham). Every 2 weeks, the urine was collected for 24 hours to measure the urinary protein excretion. Additionally, every 4 weeks, SBP was measured to examine the effect on the blood pressure.

The results were indicated by the mean ± error. In comparison between the Sham group and the 5/6Nx-Cont. group, a t-test was made without correspondence, and when the significant level is less than 5%, it is considered as a significant difference. In comparison between the 5/6Nx rat-drug groups, multiple comparison (Dunnett's test) was made respectively without correspondence following the variance analysis of one-way classification. When the significant level is less than 5%, it is considered as a significant difference.

### (Result)

### (1) Effect on urinary protein excretion

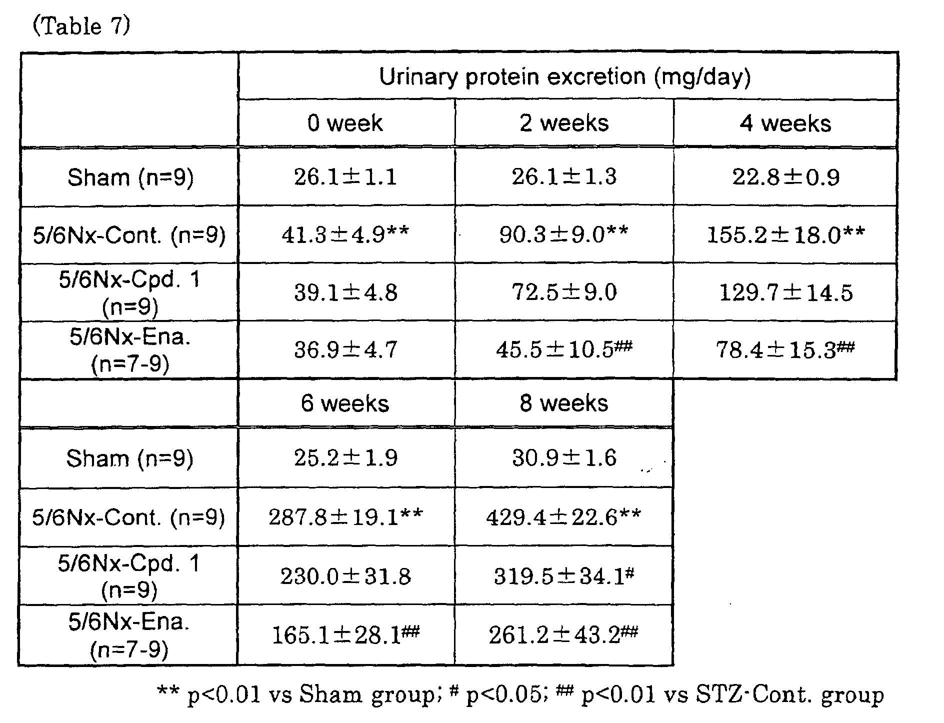

As shown in Table 7, the urinary protein excretion was significantly increased in the 5/6Nx-Cont. group more than in the Sham group. In the 5/6Nx-Ena. group, increase of the urinary protein excretion was significantly inhibited over 2 weeks to 8 weeks. In the groups to which Compound 1 was administered, increase of the urinary protein excretion was significantly inhibited at a very low dose of 1 mg/kg at the 8th week from the start of administration.

Therefore, it was suggested that Compound 1 has the effect of improving increase of the urinary protein excretion in diabetes mellitus after the progress to chronic renal failure.

### (2) Effect on elevation of blood pressure

**(Table 8)**

| | Systolic blood pressure (mmHg) | | |
|---|---|---|---|
| | 0 week | 4 weeks | 8 weeks |
| Sham (n=9) | 126±2 | 124±3 | 117±5 |
| 5/6Nx-Cont. (n=9) | 135±4 | 151±4 ** | 154±4** |
| 5/6Nx-Cpd. 1 (n=9) | 135±6 | 142±6 | 139±5 |
| 5/6Nx-Ena. (n=7-9) | 136±4 | 129±8^{#} | 124±6^{##} |

| | | | |
|---|---|---|---|
| ** p<0.01 vs Sham group; | | | |
| ^{#} p<0.05; | | | |
| ^{##} p<0.01 vs 5/6Nx-Cont. group | | | |

As shown in Table 8, SBP significantly increased in the 5/6Nx-Cont. group more than in the Sham group. In the 5/6Nx-Ena. group, a hypotensive effect was markedly recognized. In the groups to which Compound 1 was administered, no significant hypotension was observed.

### (3) Effect on the mortality rate in administration to the renal dysfunction cases

Examination was conducted on the 9 cases of the respective groups, during which time 2 death cases were confirmed in the 5/6Nx-Ena.group but no death was observed in the groups to which Compound 1 was given.

### Test Example 3: Examination on the blood pressure in spontaneous hypertensive rats (SHR)

### (Method)

Male SHRs (Hoshino Test Animal, Yashio) of 17-19 weeks of age (body weight: 276-350) were used. SHR was anesthetized with pentobarbital (60 mg/kg, i.p.) and a polyethylene cannula (PE-50) was inserted into the left common carotid artery so that the tip reached the aortic arch to fix. The other end of the cannula was pulled out from the dorsal neck and the wound part was sewed up surgically. After a certain post-operative recovering, SHR was placed in a separate cage to measure the blood pressure and heart rate under conscious and restraint. The other end of the cannula was connected to a pressure transducer (AP-601G, Nihon Kohden) through a swivel (PLYMOUTH MEETING PA, Instech Laboratories). The heart rate was measured on the pulse wave of blood pressure by driving a cardiograph (AT-601G, Nihon Kohden). The drug or solvent (0.5%MC) was given orally (5 ml/kg) through a gastric tube. SHR of which the systolic pressure was 160 mmHg or higher was used in the experiment. The results of the measurement were indicated by the mean ± error.

### (Result)

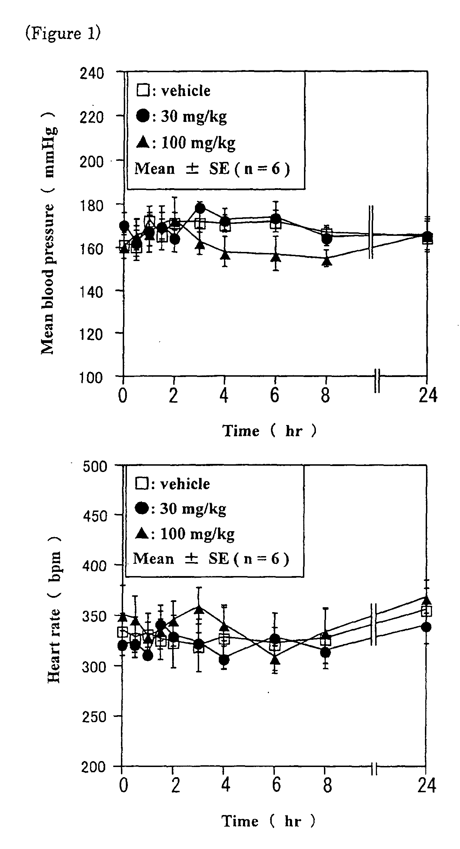

As shown in Fig. 1, when Compound 1 was orally administered at a dose of 30 mg/kg or 100 mg/kg, no effect was observed on the blood pressure and heart rate up to 24 hours after the administration in SHRs under conscious and restraint.

The current hypotensive therapy for diabetic nephropathy has an influence on the systemic hemodynamics and is in danger of decreasing renal perfusion pressure with decrease of blood pressure. On the contrary, it was suggested that Compound 1 acts in such a degree that almost no systemic blood pressure is changed and therefore it can be administered to a risky patient for whom administration of an ACE inhibitor etc. is inhibited or hesitated because there is a possibility to cause acute renal failure such as renal dysfunction.

### Test Example 4: Inhibition effect on the cell growth induced by ET-1 in rat's glomerular mesangial cells

### (Method)

### (1) Culture of rat's glomerular mesangial cells

Rat's glomerular mesangial cells were cultured according to the previously reported method (Kidney Int., 42, 860-866, 1992). That is, the kidney was taken out from a male Wistar rat of 7-10 weeks of age under anesthesia, and the glomerulus isolated aseptically by a sieving method was incubated in a condition of 95% air and 5% CO₂ at 37°C on a liquid medium of RPMI-1640 containing serum and nutritional factors to give homogeneous mesangial cells. Thereafter, the cells were sub-cultured once a week, and those of 4-10 generations were used in the experiment.

### (2) Measurement of the cell growth and hypertrophy activities

In measurement of the cell growth activity, the rat's cultured mesangial cells were incubated on a 48-well assay plate to reach 50% confluent state, at which point the nutritional factors (ITS) were removed from the culture medium and the cells were incubated for 2 days on a medium, in which the content of fetal calf serum (FCS) was lowered from 20% to 0.5%, to synchronize at the resting phase. Thereafter, the cells were incubated for 69 hours in the presence of 0.5% FCS and ITS to which ET-1, ET-3 and S6c were added. Then, Alamar Blue reagent (IWAKI)(50 ml/well) was added to the medium, which was incubated for 3 hours. The absorbance was measured at 570/600 nm and the cell number was counted from the standard curve to obtain the cell growth activity.

In measurement of the hypertrophy activity, the cells used in the measurement of the cell growth activity using an Alamar Blue reagent were washed 3 times with a phosphate buffer, to which cells was added a direct protein-determining reagent (BioRad) to measure the absorbance at 595 nm. The protein content was calculated from the analytical curve. The protein content per the unit cell number was given as the hypertrophy activity from the cell number obtained by measurement of the growth activity.

In order to examine the inhibitory effect on the growth promoting effect by ET-1, Compound 1 was added to the culture medium 30 minutes before ET-1 acting.

### (3) Statistic analysis

The results were indicated by the mean ± error. In the test of significant difference of the growth and hypertrophy activity, multiple comparison (Dunnett's test) was respectively made without correspondence following the variance analysis of one-way classification, wherein the peptide content 0 value was used as a control group. When the significant level is less than 5%, it is considered as a significant difference.

### (Result)

### (1) Effect of ET on the growth and hypertrophy of rat's cultured mesangial cells

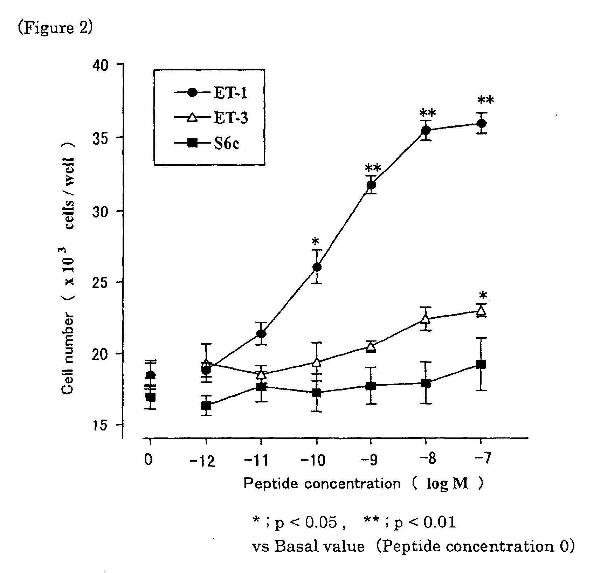

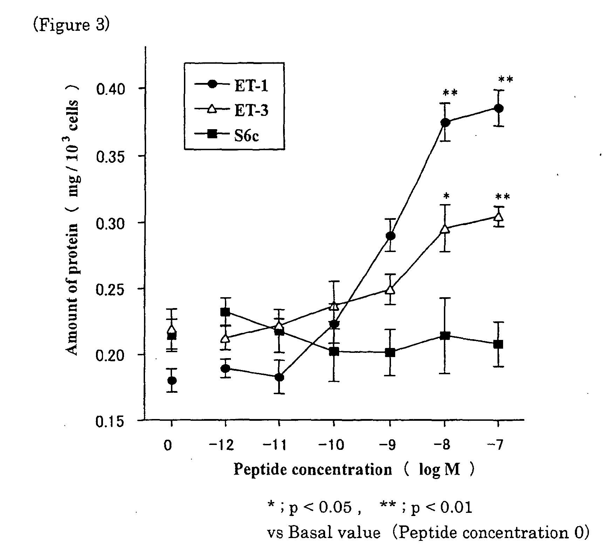

As shown in Fig.2 and Fig.3, ET-1 and ET-3 both promoted the growth and hypertrophy of rat's cultured mesangial cells depending on the concentration of 10⁻¹² to 10⁻⁷M. In both cases, the maximum reaction was obtained at 10⁻⁸M, and the growth and hypertrophy activities were more potent in ET-1 than in ET-3. There was no difference in the concentration and reaction of ET-1 and ET-3 in the growth and hypertrophy activities. Both of the reactions were considered to occur in parallel, accordingly.

### (2) Effect on the ET-1-induced growth of rat's cultured mesangial cells

**(Table 9)**

| ET-1 | Compound 1 | Cell growth (%) |
|---|---|---|
| - | - | 100.0 |
| 10⁻⁸ M | 0 | 173.3±5.8 |
| | 10⁻¹¹ M | 180.0±11.1 |
| | 10⁻¹⁰ M | 119.3±8.1 |
| | 10⁻⁹ M | 105.0±10.1 |
| | 10⁻⁸ M | 95.3±12.4 |
| (n=5-8) | | |

As shown in the above table 9, Compound 1 inhibited the growth of rat's cultured mesangial cells induced by 10⁻⁸M of ET-1 depending on the concentration of 10⁻¹¹-10⁻⁸M.

Therefore, it was suggested that Compound 1 is effective in improving ET-induced renal dysfunction accompanied by matrical change, for example, diabetic nephropathy, ET-induced early-stage nephropathy, and chronic renal failure accompanied by glomerular sclerosis which is considered as the final clinical state of complications of diabetes mellitus.

### Industrial Applicability

According to the invention, a pharmaceutical composition for improving a certain condition in diabetic patients can be provided. That is, the invention provides a pharmaceutical composition for improving elevation of the blood glucose level in a diabetic patient; a pharmaceutical composition for improving elevation of the blood lipid level in a diabetic patient after the onset of early-stage nephropathy; a pharmaceutical composition for improving renal dysfunction in a diabetic patient after the onset of early-stage nephropathy; a pharmaceutical composition for improving renal dysfunction in a diabetic patient after the progress toward chronic renal failure; a pharmaceutical composition for improving renal dysfunction in a diabetic nephropathy patient; a pharmaceutical composition for improving renal dysfunction in an ET-induced early-stage nephropathy patient; and a pharmaceutical composition for improving renal dysfunction in a patient of chronic renal failure.

Particularly, the improvement of the above-mentioned renal dysfunction can be achieved by inhibition of urinary albumin excretion caused by diabetes mellitus after the onset of early nephropathy, diabetic nephropathy and/or ET-induced early-stage nephropathy and/or inhibition of glomerular hyperfiltration, or inhibition of urinary protein excretion caused by diabetic mellitus after the progress toward chronic renal failure and/or by chronic renal failure.

The invention also provides a pharmaceutical preparation as mentioned above and/or highly orally absorbable pharmaceutical preparation as mentioned above which acts in such a degree that almost no systemic blood pressure is changed though the current hypotensive therapy for diabetic nephropathy has an influence on the systemic hemodynamics and is in danger of decreasing renal perfusion pressure with decrease of blood pressure.

## Claims

1. A pharmaceutical composition for improving one or more of specific conditions selected from the following items (1) to (7) in diabetic patients, which comprises
N-[6-methoxy-5-(2-methoxyphenoxy)-2-(pyrimidin-2-yl)pyrimidin-4-yl]-2-phenylethenesulfonamide or a pharmaceutically acceptable salt thereof as effective component:
(1) Elevation of blood glucose level
(2) Elevation of blood lipid level after the onset of early-stage nephropathy
(3) Renal dysfunction after the onset of early-stage nephropathy
(4) Increase of urinary albumin excretion after the onset of early-stage nephropathy
(5) Glomerular hyperfiltration after the onset of early-stage nephropathy
(6) Renal dysfunction after the progress toward chronic renal failure
(7) Increase of urinary protein excretion after the progress toward chronic renal failure.

2. A pharmaceutical composition as claimed in Claim 1 which is a pharmaceutical composition for improving elevation of the blood glucose level in a diabetic patient.

3. A pharmaceutical composition as claimed in Claim 1 which is a pharmaceutical composition for improving elevation of the blood lipid level in a diabetic patient after the onset of early-stage nephropathy.

4. A pharmaceutical composition as claimed in Claim 1 which is a pharmaceutical composition for improving renal dysfunction in a diabetic patient after the onset of early-stage nephropathy.

5. A pharmaceutical composition as claimed in Claim 1 which is a pharmaceutical composition for improving increase of urinary albumin excretion in a diabetic patient after the onset of early-stage nephropathy.

6. A pharmaceutical composition as claimed in Claim 1 which is a pharmaceutical composition for improving glomerular hyperfiltration in a diabetic patient after the onset of early-stage nephropathy.

7. A pharmaceutical composition as claimed in Claim 1 which is a pharmaceutical composition for improving renal dysfunction in a diabetic patient after the progress toward chronic renal failure.

8. A pharmaceutical composition as claimed in Claim 1 which is a pharmaceutical composition for improving increase of urinary protein excretion in a diabetic patient after the progress toward chronic renal failure.

9. A pharmaceutical composition for improving renal dysfunction in a diabetic nephropathy patient, which comprises N-[6-methoxy-5-(2-methoxyphenoxy)-2-(pyrimidin-2-yl)pyrimidin-4-yl]-2-phenylethenesulfonamide or a pharmaceutically acceptable salt thereof as effective component.

10. A pharmaceutical composition as claimed in Claim 9 which is a pharmaceutical composition for improving increase of urinary albumin excretion in a diabetic nephropathy patient.

11. A pharmaceutical composition as claimed in Claim 9 which is a pharmaceutical composition for improving glomerular hyperfiltration in a diabetic nephropathy patient.

12. A pharmaceutical composition for improving renal dysfunction in an ET-induced early-stage nephropathy patient, which comprises N-[6-methoxy-5-(2-methoxyphenoxy)-2-(pyrimidin-2-yl)pyrimidin-4-yl]-2-phenylethenesulfonamide or a pharmaceutically acceptable salt thereof as effective component.

13. A pharmaceutical composition as claimed in Claim 12 which is a pharmaceutical composition for improving increase of urinary albumin excretion in an ET-induced early-stage nephropathy patient.

14. A pharmaceutical composition as claimed in Claim 12 which is a pharmaceutical composition for improving glomerular hyperfiltration in an ET-induced early-stage nephropathy patient.

15. A pharmaceutical composition for improving renal dysfunction in a chronic renal failure patient, which comprises N-[6-methoxy-5-(2-methoxyphenoxy)-2-(pyrimidin-2-yl)pyrimidin-4-yl]-2-phenylethenesulfonamide or a pharmaceutically acceptable salt thereof as effective component.

16. A pharmaceutical composition as claimed in Claim 15 which is a pharmaceutical composition for improving increase of urinary protein excretion in a chronic renal failure patient.

17. A pharmaceutical composition as claimed in any one of Claims 1 to 16 which does not change the systemic blood pressure.

18. A pharmaceutical composition as claimed in any one of Claims 1 to 17 which is an oral preparation.
